# EUROPEAN PATENT SPECIFICATION

(11) **EP 0 895 777 B1**
(45) Date of publication and mention of the grant of the patent: **03.03.2004**
(21) Application number: 98114153.4
(22) Date of filing: 29.07.1998
(51) Int. Cl.: A61K 7/48, A61K 47/48, A61K 7/16, A61K 7/20, A23L 3/3526, A23L 3/3544

(54) **Compositions based on peroxyacids**
Zusammensetzungen auf Basis von Persäuren
Compositions à base de peroxyacides

(30) Priority: 05.08.1997 IT MI971882
(43) Date of publication of application: 10.02.1999
(73) Proprietor: Solvay Solexis S.p.A., 20121 Milano (IT)
(72) Inventor: Bianchi, Ugo Piero, Verona (IT); Cavallotti, Claudio, Milano (IT); Antonini, Antonella, Parma (IT)
(74) Representative: Sama, Daniele, Dr.

(56) References cited:
- EP-A- 0 441 690
- EP-A- 0 469 983
- WO-A-96/05802

## Description

The present invention relates to bleaching and hygenizing compositions having high chemical stability which is maintained during the time also after prolonged storage (shelf stability) in complex formulations, for instance in cosmetic or pharmaceutical formulations.

More particularly the invention relates to compositions to be used for preparing toothpastes or pharmaceutical compositions for the treatment of acne.

The bleaching and hygenizing properties of the organic peroxyacids, specifically of the aliphatic peroxyphthalimidic acids, such as typically the epsilon-pthalimido peroxyhexanoic acid, known as PAP, are known, see for instance EP 325,289 and EP 325,288. The reactivity of the aliphatic peroxyphthalimidic acids makes their use in various applications interesting, but it is also a technical problem, from the point of view of the setting up of formulations having high stability over time. Said organic peroxyacids indeed react very quickly so that it is not possible to prepare complex formulations stable for a long time.

The shelf-stability of the formulation foreseen for the single application is therefore an important property. To this property it is necessary to combine other essential characteristics, consistent with the different applications, taking into account the industrial safety, logistic and the general and environmental toxicology of the various formulations. Said peroxyacids have particular technical and commercial importance due to their availability on the market and to the possibility to use them in granular preparations or in dispersions.

Among the organic peroxyacids and in particular the phthalimido-peroxyalkanoic acids most important applications, there are the typical ones of the cosmetic industry, in which the peroxyacid is introduced in systems formulated with many components and must be able to be effective under particularly mild conditions and to maintain its activity during the time also after storage.

As for the toothpastes the efficacy of the peroxyacetic acid, for the setting up of formulations with improved bleaching effect is known see for intance EP 545,594.

The efficacy of phthalimido-alkanoic and/or cationic peroxyacids for the preparation of tooth systems having a bleaching activity, is also known, see for instance Patent Application WO 96/05802. In the case of the phthalimido-peroxyalkanoic acids, which have a remarkable chemical activity and bleaching and hygenizing activity, besides favourable toxicological characteristics, it is difficult to obtain unitary cosmetic formulations having polyfunctional characteristics. In this Patent Application, to prepare toothpastes, a bicomponent system formed by two complementary but separate formulations, which are mixed together at the time of use, is employed. This solution allows to maintain all the properties of the phtalimido-alkanoic peroxyacids but from the practical point of view it represents a limitation since it requires more complex equipments typical of the bicomponent systems.

The need was therefore felt to have available a formulation comprising the phthalimido-alkanoic peroxyacids as monocomponent composition.

It is known in the art to prepare adducts among peroxyacids and cyclodextrins. Specifically in EP 469,983, it is stated that the stability of the peroxycarboxylic acids adducts is greater than that of the peroxycarboxylic acid. In particular the heat-resistance, interpreted as lower tendency to uncontrolled thermal decomposition, is higher for the adducts of peroxyacids and cyclodextrins, than that of the peroxyacids as such.

Specialist technical literature is also known, see for instance D.M. Davies and M.E. Deary, "The interaction of alpha-cyclodestrin with aliphatic, aromatic and inorganic peracids, the corresponding parent acids and their respective anions" in J. Chem. Soc., Perkin Trans. 2, 1996, 2415-2421 and "Effect of alpha-cyclodextrin on the oxidation of aryl alkyl sulfides by peracids" 2423-2430; D.M. Davis et al. "Multiple pathways in the alpha-cyclodextrin catalysed reaction of iodide and substituted perbenzoic acids" in the same review 2, 1994 1531-1537. In these publications the specific chemical interaction between alpha-cyclodextrins and aliphatic, aromatic and inorganic peroxyacids, the reactivity and the nature of the chemical adducts obtained by interaction of the two components, are reported. From said publications it appears how the reactivity of the peroxyacids results marginally modified in the adducts with reference to the peroxyacids as such. The typical oxidation reactions of the iodide ion and of the Rₐ-S-Rₐ' group are reported, wherein Rₐ and Rₐ' are alkyls and/or aryls equal to or different from each other, in aqueous phase with the adducts and with the peroxyacids as such. These reactions occur in the adduct case in mild conditions, sometimes with even higher easiness with reference to the peroxyacids as such. This means that the reactivity of the peroxyacids present in the adducts is higher than that of the peroxyacids as such, See for instance the data reported for the proxyacetic acid and for its adduct. The adducts therefore do not represent a chemically advantageous form to obtain formulations stable during the time.

The need was felt to have available complex formulations stable during the time of industrial interest in which the peroxyacid can be introduced without loosing its reactivity characteristics.

A process for preparing adducts among cyclodextrins, specifically betacyclodextrins, and organic peroxides selected from dialkyl- and diacyl-peroxides is also known, which find interest in the cosmetic product formulation. See for instance EP 441,690. It is a process based on the dispersion and the contact of the two components in water (80 parts) and ethanol (20 parts) mixtures, at temperatures in the range from 40° to 64°C.

It has been surprisingly and unexpectedly found that it is possible to prepare complex formulations comprising phthalimido-proxycarboxylic acids, in particular the epsilon-phthalimido-peroxyhexanoic acid, having high stability during the time and maintaining the typical proxyacid reactivity. This unexpected fact has allowed to prepare the above mentioned monocomponent formulations, with all the advantages resulting therefrom, for cosmetic, pharmaceutical and food applications.

An object of the present invention is use of phthalimido-peroxycarboxylic acids, preferably of the epsilon-phthalimido-peroxyhexanoic acid, under the adduct form with cyclodextrins, preferably beta-cyclodextrins, for the preparation of cosmetic, pharmaceutical and food formulations.

The molar ratio between the invention peroxyacids and the cyclodextrins is in the range 1:1-1:2.

The imido-alkanpercarboxylic acids which can be used according to the present invention have the general formula: 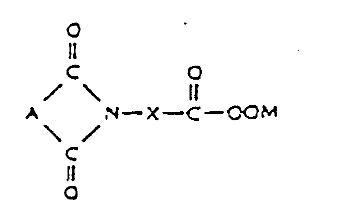 wherein A indicates one group having the formula 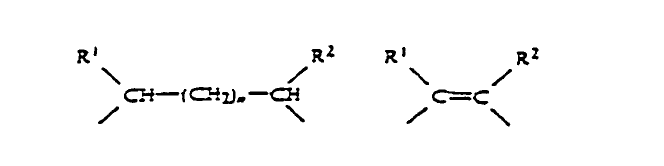 or 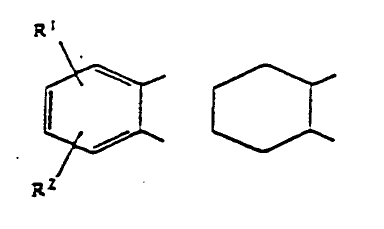 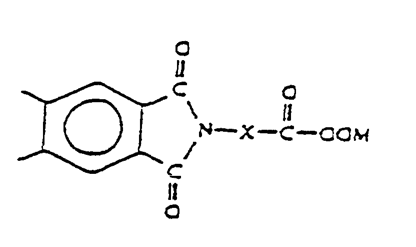 n is an integer 0, 1 or 2,
R¹ is hydrogen, chlorine, bromine, C₁-C₂₀ alkyl,
C₂-C₂₀ alkenyl, aryl or alkylaryl,
R² is hydrogen, chlorine, bromine or one group having the formula -SO₃M, -CO₂M, -CO₃M, -OSO₃M,
M indicates hydrogen, an alkaline metal or ammonium ion or the equivalent of an earth-alkaline metal ion,
and X indicates C₁-C₁₉ alkylene or arylene;
Y indicates a C₃-C₁₉ alkylene.

Among said imidoalkanpercarboxylic acids the epsilon-phthalimidoperoxyhexanoic acid is particularly preferred.

Among the cyclodextrins we can mention the alpha, beta, gamma or delta cyclodextrins, the modified alpha, beta, gamma or delta cyclodextrins with acylic, alkylic, hydroxyalkylic groups, generally having from 1 to 10 carbon atoms, preferably from 2 to 6; the mixtures of said cyclodextrins and the mixtures of said cyclodextrins and of dextrins or linear oligosaccharides, beta-cyclodextrins are preferably used.

The peroxidic activity of such adducts, where there is the active oxygen typical of the free peroxyacid, is modified so to render possible to broaden the field of technical applicability of the peroxyacid itself to multicomponent systems typical of the cosmetic industry, wherein the free peroxyacid would not in practice be usable, due to chemical incompatibility.

A further object of the invention is a process for preparing peroxyacid adducts and in particular the epsilon-phthalimido peroxyhexanoic acid, and the cyclodextrins and in particular the beta cyclodextrins, which foresees the direct contact of the two components dispersed in an aqueous phase at room temperature, under continuous stirring for a time ranging from 2 to 12 hours, preferably from 6 to 8 hours. After said time the aqueous dispersion is filtered and the solid is recovered and dried by the methods known in the art, preferably by treatment with dry air at moderate tempratures, preferably between 20° and 50°C. The above described process is of particular industrial interest since high temperatures are not used, room temperature and inexpensive and easily available equipments is preferable.

The cosmetic formulations comprise the well known typical components, for instance surfactants, among which we can mention octyloctonate, emollients such as glycerine, dispersants, oils and greases, perfumed essences, optionally fillers such as micronized silica, oxides such as for instance titanium oxide, etc. The amount of adduct is generally in the range of 1-20% and preferably of 5-10%.

The pharmaceutical formulations comprise the known pharmaceutical excipients and an amount of adduct generally in the range of 1-30% by weight and preferably of 10-20%.

The food formulations comprise the known food excipients and an adduct amount generally in the range of 1-10% by weight and preferably of 2-5%.

The application of the peroxyacid adducts and in particular of the epsilon-phthalimido peroxyhexanoic acid, and the cyclodextrins and in particular the beta cyclodextrins in simple or complex systems of cosmetic, pharmaceutical and food importance, is of remarkable interest for the operators skilled in the art.

Some examples are hereinafter reported for illustrative but not limitative purposes of the present invention.

### EXAMPLE 1

5,650 g of beta cyclodextrin are added at room temperature to an aqueous suspension of 1,856 g of epsilon-phthalimido-peroxyhexanoic acid (water content 17% by weight; title 93% by weight) in 16 1 of demineralized water, maintained under continuous stirring.

After the addition, the stirring is continued for 12 hours, keeping the system at room temperature (about 20°C). The obtained pulp is then filtered, and the solid is recovered. After drying that in an air-circulation stove at 50°C for 96 hours, about 7 kg of adduct are thus obtained, with an active oxygen content of 1.1% by weight.

### EXAMPLE 2

297 g of beta-cyclodextrin and 46 g of wet epsilon-phthalimido-peroxyhexanoic acid (water content 17% by weight; title 95% by weight) are suspended in 800 ml of demineralized water at 30°C, by maintaining the system under stirring for 8 hours.

After filtering and drying (as in case 1) 320 g of adduct with an active oxygen content of 0.6% by weight are obtained.

The thermal stability of the adduct results in a peroxidic loss of 0.3%, after 3 days in an air-circulation stove at 50°C.

### EXAMPLE 3

38 g of dry epsilon-phthalimido-peroxyhexanoic acid (title 95% by weight) are added with 148 g of beta-cyclodextrin to 400 ml of demineralized water, maintained at room temperature and under stirring. Stirring is continued for 12 hours. After drying, 175 g of adduct with an active oxygen content of 1.1% by weight, is obtained.

### EXAMPLE 4

### Tooth formulation

10 g of adduct among beta-cyclodextrins and epsilon-phthalimido-peroxyhexanoic acid, obtained as in Example 1, are mixed to 200 g of a base toothpaste having a standard composition (in % by weight):

| | |
|---|---|
| glycerine | 35 % |
| water | 33 % |
| hydrate silica | 23 % |
| propylene glycol | 3 % |
| sodium laurylsulphate | 1.25 % |
| tetrasodium pyrophosphate | 1.2 % |
| carboxymethylcellulose | 1.2 % |
| perfumes | 1 % |
| titanium oxide | 0.5 % |
| sweeteners | 0.3 % |
| trichlosan | 0.3 % |
| sodium fluoride | 0.25 % |

The obtained paste is manufactured in a plasticized container and placed in an air-circulation stove, where it is maintained for 60 days at 37°C.

The noted peroxidic loss was of 5.7%.

For comparison, the peroxidic loss noted in the case of a corresponding product obtained by adding to 200 g of the standard tooth base 2.25 g of dry epsilon-phthalimido-peroxyhexanoic acid (title 95% by weight) and operating in a similar way, was higher than 95% by weight.

### EXAMPLE 5

### Cosmetic cream

10 g of adduct among beta-cyclodextrins and epsilon-phthalimido-peroxyhexanoic acid, obtained as in Example 1, are mixed with 200 g of a base cream having a standard composition:

| | |
|---|---|
| cetearil glucoside | 5.0 % |
| octyl octanoate | 14.0 % |
| C12/C15-octyl-octanoate | 12.0 % |
| Butyrospermum parkii | 3.0 % |
| dimethicone | 2.0 % |
| dimethiconol-dimethicone | 1.5 % |
| oryzanol | 0.5 % |
| tocopherol/lecithin/ascorbyl palmitate/citric acid | 0.08 % |
| water | 59.32 % |
| potassium cetyl phosphate | 2.0 % |
| imidazolidinyl urea | 0.25 % |
| methylchloroisothiazolinone/methylisothiazolinone | 0.15 % |
| disodium EDTA | 0.20 % |

The obtained cream is manufactured in a plasticized container and placed in an air-circulation stove, where it is maintained for 60 days at 37°C.

The noted peroxidic loss was lower than 10%, in comparison with a peroxidic loss higher than 98% noted for the corresponding system obtained by mixing to 200 g of the same base cream 2.5 g of dry epsilon-phthalimido-peroxyhexanoic acid (title: 95%).

## Claims

1. Use of epsilon-phthalimidoperoxyhexanoic acid, in the form of adducts with cyclodextrins for preparing cosmetic, pharmaceutical and food formulations.

2. Use of phthalimido-peroxycarboxylic acids according to claim 1, wherein the cyclodextrins are selected from alpha, beta, gamma or delta cyclodextrins; modified alpha, beta, gamma or delta cyclodextrins with acylic, alkylic, hydroxyalkylic groups; mixtures of said cyclodextrins and mixtures of said cyclodextrins and dextrins or linear oligosaccharides, preferably beta-cyclodextrins.

3. Use of phthalimido-peroxycarboxylic acids according to claim 1 to 2, wherein the peroxyacid and the cyclodextrin molar ratio is in the range of 1:1-1:2.

4. Cosmetic formulations comprising the adduct according to claims from 1 to 3.

5. Cosmetic formualtions according to claim 4 for preparing toothpastes.

6. Pharmaceutical formulations comprising the adduct according to claims from 1 to 3.

7. Pharmaceutical formulations according to claim 6 for the treatment of acne.

8. Food formulations comprising the adduct according to claims from 1 to 3.

9. Adducts of epsilon-phtalimido-peroxyhexanoic acid with cyclodextrins according to claims from 1 to 4.

10. Process for preparing adducts according to claim 9 which foresees the direct contact of the two components dispersed in an aqueous phase at room temperature, under continuous stirring for a time ranging from 2 to 12 hours, preferably from 6 to 8 hours; the filtering and the recovery of the solid, its drying, preferably by dry air teatment at moderate temperatures, preferably in the range 20°-50°C.

## Patentansprüche

1. Verwendung von epsilon-Phthalimidoperoxyhexansäure in der Form von Addukten mit Cyclodextrinen zum Herstellen von kosmetischen, pharmazeutischen und Nahrungsmittelformulierungen.

2. Verwendung von Phthalimidoperoxycarbonsäuren gemäß Anspruch 1, wobei die Cyclodextrine ausgewählt sind aus alpha-, beta-, gamma- oder delta-Cyclodextrinen; modifizierten alpha-, beta-, gamma- oder delta-Cyclodextrinen mit Acyl-, Alkyl-, Hydroxyalkylgruppen; Mischungen aus diesen Cyclodextrinen und Mischungen aus diesen Cyclodextrinen und Dextrinen oder linearen Oligosacchariden, vorzugsweise beta-Cyclodextrinen.

3. Verwendung von Phthalimidoperoxycarbonsäuren gemäß Anspruch 1 bis 2, wobei das molare Verhältnis von Peroxysäure zum Cyclodextrin im Bereich von 1:1-1:2 liegt.

4. Kosmetische Formulierungen, umfassend das Addukt gemäß Anspruch 1 bis 3.

5. Kosmetische Formulierungen gemäß Anspruch 4 zum Herstellen von Zahnpasta.

6. Pharmazeutische Formulierungen, umfassend das Addukt gemäß Anspruch 1 bis 3.

7. Pharmazeutische Formulierungen gemäß Anspruch 6 für die Behandlung von Akne.

8. Nahrungsmittelformulierungen, umfassend das Addukt gemäß Anspruch 1 bis 3.

9. Addukte von epsilon-Phthalimidoperoxyhexansäure mit Cyclodextrinen gemäß Anspruch 1 bis 4.

10. Verfahren für die Herstellung von Addukten gemäß Anspruch 9, welches den direkten Kontakt der zwei Bestandteile, die in einer wäßrigen Phase bei Raumtemperatur dispergiert sind, unter kontinuierlichem Rühren für eine Zeitperiode von 2 bis 12 Stunden, vorzugsweise von 6 bis 8 Stunden, vorsieht; anschließend Filtern und Rückgewinnung des Feststoffes, dessen Trocknung, vorzugsweise durch Behandlung mit Trockenluft bei moderaten Temperaturen, vorzugsweise im Bereich von 20°-50°C.

## Revendications

1. Utilisation de l'acide epsilon-phtalimido-peroxyhexanoïque sous forme d'un complexe d'inclusion avec des cyclodextrines pour la préparation de compositions cosmétiques, pharmaceutiques et alimentaires.

2. Utilisation d'acide phtalimido-peroxyhexanoïque selon la revendication 1, dans laquelle les cyclodextrines sont choisies parmi les cyclodextrines α, β, γ ou δ, les cyclodextrines α, β, γ ou δ modifiées par des groupes acyle, alkyle ou hydroxyalkyle, les mélanges desdites cyclodextrines entre elles et les mélanges desdites cyclodextrines avec des oligosaccharides linéaires, de préférence les β-cyclodextrines.

3. Utilisation d'acide phtalimido-peroxyhexanoïque selon la revendication 1 ou 2, dans laquelle le rapport molaire du peroxyacide à la cyclodextrine est compris entre 1:1 et 1:2.

4. Composition cosmétique comprenant le complexe d'inclusion selon l'une des revendications 1 à 3.

5. Composition cosmétique selon la revendication 4, pour la préparation de pâtes dentifrice.

6. Composition pharmaceutique comprenant le complexe d'inclusion selon les revendications 1 à 3.

7. Composition pharmaceutique selon la revendication 6 pour le traitement de l'acné.

8. Composition alimentaire comprenant le complexe d'inclusion selon les revendications 1 à 3.

9. Complexe d'inclusion d'acide epsilon-phtalimido-peroxyhexanoïque avec des cyclodextrines selon les revendications 1 à 4.

10. Procédé de préparation de complexes d'inclusion selon la revendication 9 qui comprend la mise en contact directe des deux composants dispersés dans une phase aqueuse à température ambiante et sous agitation continue pendant une durée comprise entre 2 et 12 heures, de préférence entre 6 et 8 heures, la filtration et la récupération du solide, son séchage, de préférence par un traitement avec de l'air sec à des températures modérées, de préférence comprises entre 20 °C et 50 °C.
